# EUROPEAN PATENT APPLICATION

(11) **EP 3 210 645 A1**
(43) Date of publication of application: **30.08.2017**
(21) Application number: 17156480.0
(22) Date of filing: 16.02.2017
(51) Int. Cl.: A61M 25/00

(54) **CATHETER**

(30) Priority: 22.02.2016 JP 2016030691
(71) Applicant: Asahi Intecc Co., Ltd., Nagoya-shi, Aichi 463-0024 (JP)
(72) Inventor: Ishikawa, Masatomo, Nagoya-shi, Aichi 463-0024 (JP)
(74) Representative: Winter, Brandl, Fürniss, Hübner, Röss, Kaiser, Polte - Partnerschaft mbB

(57) **Abstract**

To provide a catheter allowing injection of a contrast medium or the like and securing of a blood flow at an arbitrary position on the entire circumference of the catheter while certainly securing lumina for injecting a contrast medium or the like or securing a blood flow. A catheter having a proximal end and a distal end includes a hollow structure forming a plurality of lumina (5, 3, 33, 43) extending in a direction from the proximal end to the distal end. The hollow structure includes a first lumen (5) formed in the center of the hollow structure, and a plurality of second lumina (3, 33, 43) formed along a circumferential direction around the first lumen, wherein each of the plurality of second lumina is in communication with the outside of the catheter by at least one hole (15, 35, 45, 55).

## Description

### Technical Field

The present invention relates to a catheter that is inserted into a lumen such as a blood vessel for use.

### Background

Conventionally, there is known a catheter inserted into a lumen such as a blood vessel for use, and physicians performing procedures have inserted a guide wire into a lumen of a catheter and have controlled the catheter to reach a lesion part, or have disposed devices such as a stent and an embolic coil at the lesion part via the lumen of the catheter. Moreover, the physicians have injected, using a catheter having a side hole on the surface thereof, a medical liquid or a contrast medium into a blood vessel through the side hole, or have secured a blood flow through the side hole when the catheter is left in a blood vessel and the like.

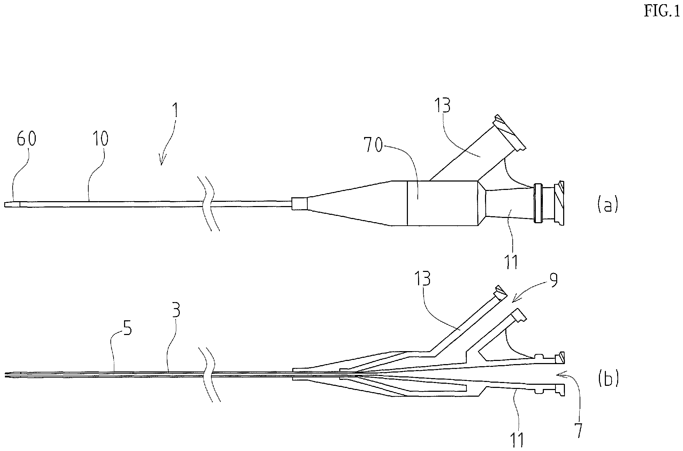

For example, Patent Literature 1 describes the triple-lumen catheter 1 including the first lumen 8, the second lumen 7, and the third lumen 9 (see FIG. 1, and the like), and the third lumen 9 of the triple-lumen catheter 1 has a side hole 10 (see paragraph [0020], FIG. 1, and the like). Patent Literature 1 also describes that in the triple-lumen catheter 1, the third lumen communicates with the outside through the side hole 10 (see paragraph [0019], FIG. 1, and the like).

In addition, Patent Literature 1 describes the triple-lumen catheter main body 22, in which the blood removal lumen 25 has two side holes 25a.

### Citation List

### Patent Literature

Patent Literature 1: Japanese Patent Application Laid-open No. 2006-346183

### Summary

### Technical Problem

Meanwhile, when a contrast medium or the like is injected or when a blood flow is secured, it is considered to be more efficient that the catheter has side holes on the entire circumference thereof.

However, in the above-described conventional catheter including a plurality of lumina, no lumen is formed on the entire circumference of the catheter, and thus the position of a side hole formed on a lumen is restricted. Therefore, when a contrast medium or the like is injected or when a blood flow is secured, it has been difficult to inject a contrast medium or the like or secure a blood flow at an arbitrary position on the entire circumference of the catheter.

In addition, in order to inject a contrast medium or the like or secure a blood flow at an arbitrary position on the entire circumference of the catheter, it has been necessary to consider securing of a lumen for injecting a contrast medium or the like or securing a blood flow.

In view of the above-described problems of the conventional technology, the present invention aims at providing a catheter capable of injecting a contrast medium or the like and securing a blood flow at an arbitrary position on the entire circumference of the catheter while certainly securing a lumen for injecting a contrast medium or the like or securing a blood flow.

### Solution to Problem

In order to solve the above-described problems, a catheter according to a first aspect of the invention includes a hollow structure forming a plurality of lumina extending in a direction from the proximal end to the distal end of the catheter. The hollow structure includes a first lumen formed in the center of the hollow structure, and a plurality of second lumina formed along a circumferential direction around the first lumen, wherein each of the plurality of second lumina is in communication with the outside of the catheter by at least one hole.

That is, in the catheter of the first aspect, on the outer periphery of the catheter, a plurality of holes are formed along the circumferential direction of the catheter, so as to inject a contrast medium or the like from the holes and secure a blood flow through the holes. Here, the hollow structure includes the second lumina formed along a circumferential direction around the first lumen. The plurality of second lumina are separated from each other and each of second lumina is in communication with the outside of the catheter by at least one hole. This makes it possible to inject a contrast medium or the like and secure a blood flow at an arbitrary position of the entire circumference of the catheter while certainly securing lumina for injecting a contrast medium or the like and securing a blood flow.

Moreover, the catheter according to the second aspect of the invention is the catheter of the first aspect, wherein the hollow structure is formed of a first hollow body that forms the first lumen; and a second hollow body that covers an outer periphery of the first hollow body and is connected to the first hollow body by a plurality of partition walls extending in the direction from the proximal end to the distal end, to form the plurality of second lumina between the first hollow body and the second hollow body. Each of the plurality of second lumina is in communication with the outside of the catheter by at least one hole penetrating the second hollow body.

Moreover, the catheter according to the third aspect of the invention is the catheter of the first aspect, wherein the hollow structure is formed of a hollow body that forms the first lumen and a plurality of tube bodies disposed along a longitudinal direction of the catheter on the outer periphery of the hollow body to form the plurality of second lumina. Each of the plurality of second lumina is in communication with the outside of the catheter by at least one hole penetrating a respective one of the tube bodies.

Furthermore, the catheter according to the fourth aspect of the invention is the catheter of the third aspect, in which the tube bodies are helically wound around the outer periphery of the hollow body.

### Advantageous Effects of Invention

The catheter according to the first and second aspect of the invention, it is possible to inject a contrast medium or the like at an arbitrary position of the entire circumference of the catheter and secure a blood flow while certainly securing lumina for injecting a contrast medium or the like and securing a blood flow.

In the catheter according to the third aspect of the invention, the plurality of second lumina are formed by disposing a plurality of tube bodies on the outer periphery of the hollow body, and the hole is formed for each of the tube bodies. This exerts the effect of preventing kinks of the second lumina, in addition to the effect of the catheter of the first and second aspects.

Moreover, with the use of the tube bodies forming the second lumina, the manufacture of the catheter is easier. Thus, it is possible to easily achieve injection of a contrast medium or the like at an arbitrary position of the entire circumference of the catheter and securing of a blood flow while certainly securing lumina for injecting a contrast medium or the like and securing a blood flow.

Furthermore, in the catheter according to the fourth aspect of the invention, a plurality of tube bodies are wound helically around the outer periphery of the first hollow body. This exerts the effect of improving flexibility of the catheter in addition to the effect of the catheter of the second aspect.

Moreover, the tube bodies can be easily twisted using a conventional twisting machine. Thus, it is possible to more easily achieve injection of a contrast medium or the like at an arbitrary position of the entire circumference of the catheter and securing of a blood flow while certainly securing lumina for injecting a contrast medium or the like and securing a blood flow.

### Brief Description of Drawings

FIG. 1 is a diagram illustrating an overview of a catheter according to a first embodiment of the present invention, in which FIG. 1(a) is a general view and FIG. 1(b) is a general longitudinal section view.
FIG. 2 is a distal end enlarged view of FIG. 1(b).
FIG. 3 is a cross section view along A-A of FIG. 2.
FIG. 4 is a diagram illustrating an overview of a catheter according to a second embodiment.
FIG. 5 is a cross section view along B-B of FIG. 4.
FIG. 6 is a diagram illustrating an overview of a catheter according to a third embodiment.
FIG. 7 is a cross section view along C-C of FIG. 6.
FIG. 8 is a diagram illustrating an overview of a catheter according to a fourth embodiment.

### Description of Embodiments

The following will describe embodiments of the catheter of the present invention with reference to the enclosed drawings.

### (First embodiment)

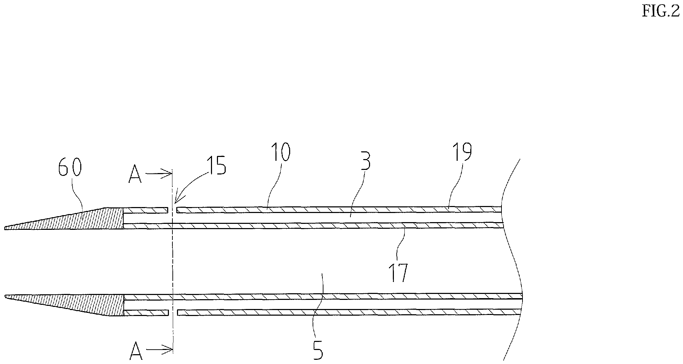

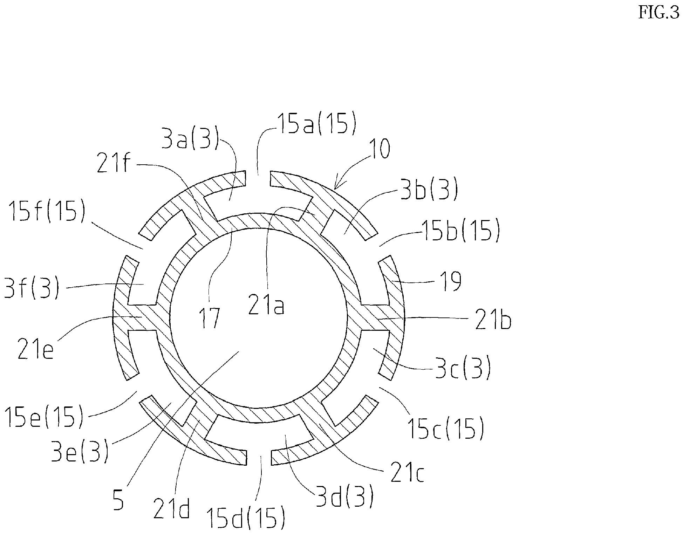

FIG. 1 is a diagram illustrating an overview of a catheter according to the first embodiment of the present invention, in which FIG. 1(a) is a general view and FIG. 1 (b) is a general longitudinal section view. FIG. 2 is a distal end enlarged view of FIG. 1(b), and FIG. 3 is a cross section view along A-A of FIG. 2.

As illustrated in FIG. 1(a), a catheter 1 of the embodiment includes a catheter shaft 10, a distal end tip 60 provided at a distal end of the catheter shaft 10, and a connector 70 provided at a proximal end of the catheter shaft 10.

As illustrated in FIG. 1(b), the catheter shaft 10 has a hollow shaped multiple-lumina structure including a first lumen 5 extending in the axial direction of the catheter shaft 10 in the center of the cross section and a plurality of second lumina 3 formed on the outer periphery of the first lumen 5 and extending in the axial direction of the catheter shaft 10.

The connector 70 includes a first connector 11 with a first opening 7 through which a guide wire or the like is inserted, and a second connector 13 with a second opening 9 to which a syringe (not illustrated) for injecting a contrast medium or the like is connected. The first opening 7 communicates with the first lumen 5, and the second opening 9 communicates with the second lumina 3.

Moreover, as illustrated in FIG. 2, the catheter shaft 10 includes a first hollow body 17, a second hollow body 19, and partition walls 21a to 21f (see FIG. 3).

On the outer periphery of the catheter shaft 10, a plurality of holes 15 are formed in the vicinity of a joint between the catheter shaft 10 and the distal end tip 60. In the embodiment, six holes 15 in total (holes 15a to 15f) are formed in the circumferential direction of the catheter shaft 10 with intervals of about 60° of a central angle, as illustrated in FIG. 3.

Here, when a contrast medium or the like is injected from a syringe connected to the opening 9, the contrast medium or the like diverges to the second lumina 3a to 3f (3) divided by the partition walls 21a to 21f (see FIG. 3) formed in the catheter shaft 10, and is discharged from the six holes 15a to 15f (15).

Note that a resin material forming the distal end tip 60 is not particularly limited. The embodiment considers a risk of damages of blood vessels, and uses polyurethane resin that is relatively flexible.

The catheter of the embodiment includes the first hollow body 17 that has the first lumen 5, and the second hollow body 19 that covers an outer periphery of the first hollow body 17 and has the second lumina 3 between the first hollow body 17 and the second hollow body 19, in which the second lumina 3 are separated from each other by a plurality of partition walls 21a to 21f connecting the first hollow body 17 and the second hollow body 19, and the holes 15 penetrating the second hollow body 19 are formed along a circumference direction of the second hollow body. Each of the holes 15 is formed for one of the second lumina 3 separated by the partition walls 21a to 21f. Thus, it is possible to inject a contrast medium or the like at an arbitrary position of the entire circumference of the catheter 1 and secure a blood flow while certainly securing lumina for injecting a contrast medium or the like and securing a blood flow.

### (Second embodiment)

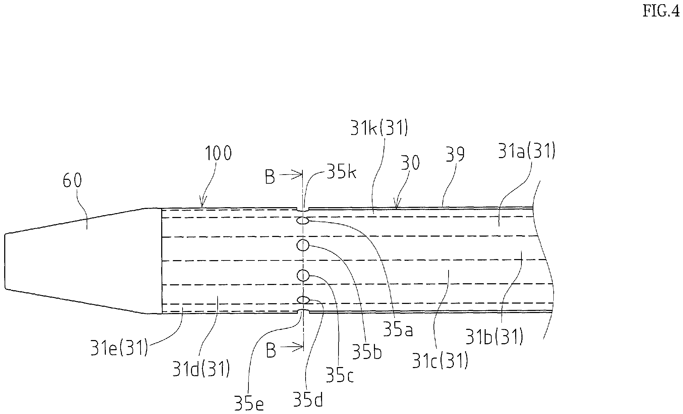

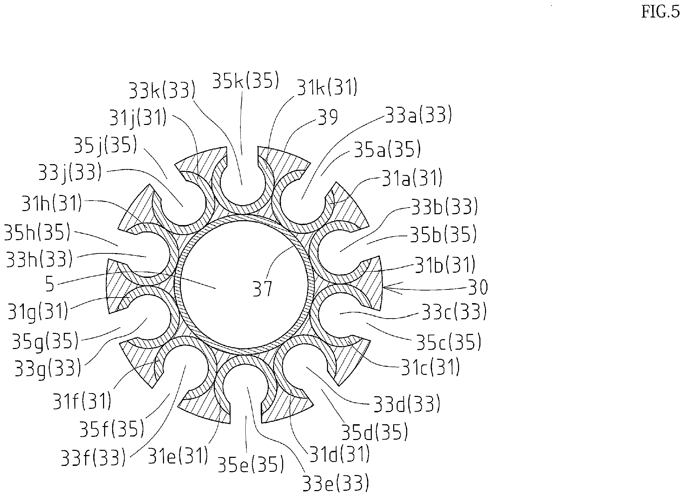

FIG. 4 is a diagram illustrating an overview of a catheter according to the second embodiment, and FIG. 5 is a cross section view along B-B of FIG. 4.

A catheter 100 of the second embodiment is different from the above-described catheter 1 of the first embodiment in the following point. That is, in the catheter 1 of the first embodiment, the first hollow body 17, the second hollow body 19, and a plurality of partition walls 21a to 21f form the lumina 3a to 3f, and the holes 15a to 15f are formed for the respective lumina. Meanwhile, in the catheter 100 of the second embodiment, a plurality of tube bodies 31 are disposed along a longitudinal direction of the catheter on the outer periphery of a hollow body 37. An outer layer 39 covers an outer periphery of the tube bodies 31, and a hole 35 is formed for each of the tube bodies 31.

As illustrated in FIG. 4, a catheter shaft 30 includes the hollow body 37, a plurality of tube bodies 31 provided on the outer periphery of the hollow body 37, and the outer layer 39 covering a plurality of the tube bodies 31.

On the outer periphery of the catheter 100, a plurality of holes 35 are formed in the vicinity of a joint between the catheter shaft 30 and the distal end tip 60. In the embodiment, 10 holes 35 in total (holes 35a to 35k) are formed in the circumferential direction of the catheter 100 with intervals of about 36° of a central angle, as illustrated in FIG. 5.

Here, when a contrast medium or the like is injected from a syringe connected to the opening 9, it diverges to lumina 33a to 33k (33) divided by the tube bodies 31a to 31k (see FIG. 5) formed in the catheter 100, and is discharged from the 10 holes 35a to 35k (35).

Note that the tube bodies 31 are formed of resin, and, in the embodiment, they are formed of polyether ketone (PEEK) resin. The PEEK resin has a melting point of about 330°C, and is known as resin having a relatively high heat-resistant property for thermoplastic resin allowing injection molding.

Moreover, the outer layer 39 is also formed of resin, and polyamide, polyamide elastomer, polyester, polyurethane, and the like can be used, for example, without any limitation thereto.

According to the catheter of the embodiment, a plurality of tube bodies 31a to 31k (10 tube bodies in the embodiment) are formed on the circumference of the hollow body 37, and each of the holes 35a to 35k (35) is formed for the respective one of the tube bodies 31a to 31k. This exerts the effect of preventing kinks of lumina to which a contrast medium or the like is injected, in addition to the effect of the catheter 1 of the first embodiment.

Moreover, with the use of the tube bodies 31, the manufacture of the catheter is easier. Then, it is possible to easily achieve injection of a contrast medium or the like at an arbitrary position of the entire circumference of the catheter and securing of a blood flow while certainly securing lumina for injecting a contrast medium or the like and securing a blood flow.

### (Third embodiment)

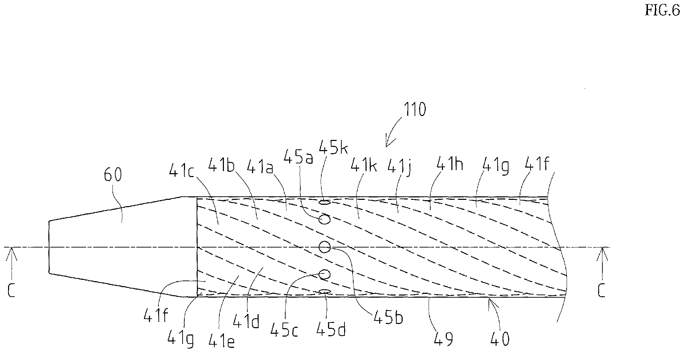

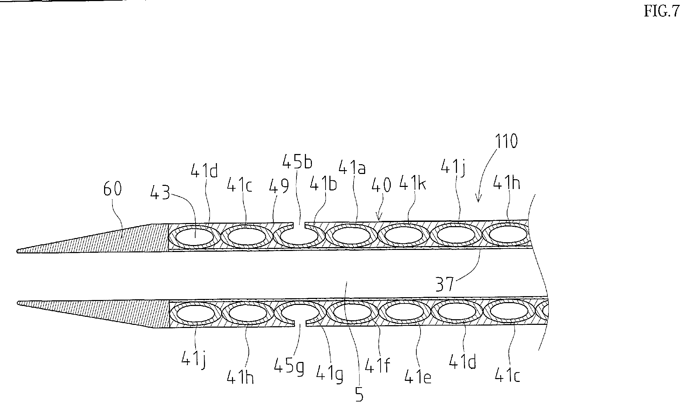

FIG. 6 is a diagram illustrating an overview of a catheter according to the third embodiment, and FIG. 7 is a cross section view along C-C of FIG. 6.

A catheter 110 of the third embodiment is different from the above-described catheter 100 of the second embodiment in the following point. That is, in the catheter 100 of the second embodiment, a plurality of tube bodies 31 are disposed along a longitudinal direction of the catheter on the outer periphery of the hollow body 37. Meanwhile, in the catheter 110 of the third embodiment, a plurality of tube bodies 41 (41a to 41k) are helically wound around the outer periphery of the hollow body 37, and a hole 45 (45a to 45k) is formed for each of the tube bodies 41.

As illustrated in FIG. 6, a catheter shaft 40 includes the hollow body 37, a plurality of tube bodies 41 disposed on the outer periphery of the hollow body 37, and an outer layer 49 covering a plurality of the tube bodies 41.

On the outer periphery of the catheter 110, a plurality of holes 45 are formed in the vicinity of a joint between the catheter shaft 40 and the distal end tip 60. In the embodiment, 10 holes 45 in total (holes 45a to 45k) are formed in the circumferential direction of the catheter 110 with intervals of about 36° of a central angle.

Here, when a contrast medium or the like is injected from a syringe connected to the opening 9, it diverges to lumina 43 divided by the tube bodies 41a to 41k formed in the catheter 110, and is discharged from the 10 holes 45a to 45f (45).

Note that the tube bodies 41 are formed of polyether ketone (PEEK) resin, similarly to the above-described tube bodies 31. Also for the outer layer 49, the same resin as for the above-described outer layer 39 can be used.

According to the catheter of the embodiment, a plurality of tube bodies 41 are helically wound around the outer periphery of the hollow body 37. This exerts the effect of improving flexibility of the catheter, in addition to the effect of the catheter 100 of the second embodiment.

Moreover, in the catheter of the embodiment, the tube bodies 41 can be easily wound around the outer periphery of the hollow body 37 using a conventional twisting machine. Thus, it is possible to more easily achieve injection of a contrast medium or the like at an arbitrary position of the entire circumference of the catheter and securing of a blood flow while certainly securing lumina for injecting a contrast medium or the like and securing a blood flow.

### (Fourth embodiment)

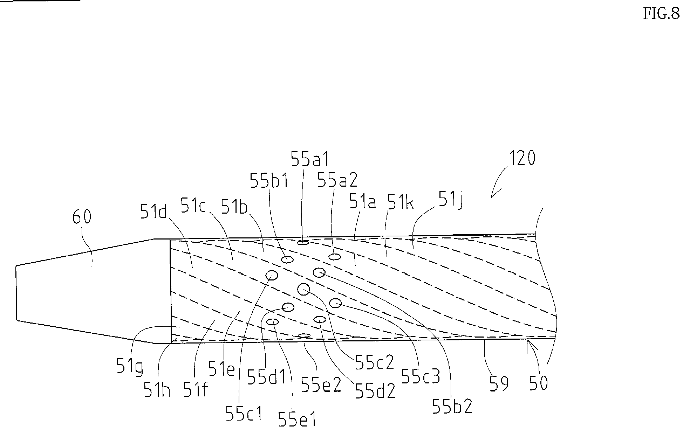

FIG. 8 is a diagram illustrating an overview of a catheter according to a fourth embodiment.

A catheter 120 of the fourth embodiment is different from the above-described catheter 110 of the third embodiment in the following point. That is, in the catheter 110 of the third embodiment, the single hole 45 is formed for each of the tube bodies 41 helically wound. Meanwhile, in the catheter 120 of the fourth embodiment, a plurality of holes 55 are formed for each of tube bodies 51 helically wound.

As illustrated in FIG. 8, a catheter shaft 50 includes the hollow body 37, a plurality of tube bodies 51 provided on an outer periphery of the hollow body 37, and an outer layer 59 covering plurality of the tube bodies 51.

Moreover, on the outer periphery of the catheter 120, a plurality of holes 55 (55a1, 55a2, 55b1, 55b2, 55c1, 55c2, 55c3, 55d1, 55d2, 55e1, and 55e2) are formed in the vicinity of a joint between the catheter shaft 50 and the distal end tip 60.

Here, when a contrast medium or the like is injected from a syringe connected to the opening 9, it diverges to lumina 53 (not illustrated) divided by the tube bodies 51a to 51k formed in the catheter 120, and is discharged from the holes 55.

Note that the tube bodies 51 are formed of polyether ketone (PEEK) resin, similarly to the above-described tube bodies 31 and tube bodies 41. Also for the outer layer 59, the same resin as for the above-described outer layer 39 and outer layer 49 can be used.

### Reference Signs List

- 1, 100, 110, 120: catheter
- 3, 33, 43: second lumen
- 5: first lumen
- 10, 30, 40, 50: catheter shaft
- 17: first hollow body
- 19: second hollow body
- 31, 41, 51: tube body
- 15, 35, 45, 55: hole
- 37: hollow body
- 39, 49, 59: outer layer
- 60: distal end tip
- 70: connector

## Claims

1. A catheter having a proximal end and a distal end, comprising:
a hollow structure forming a plurality of lumina (5, 3, 33, 43) extending in a direction from the proximal end to the distal end and including:
a first lumen (5) formed in the center of the hollow structure, and
a plurality of second lumina (3, 33, 43) formed along a circumferential direction around the first lumen (5),
wherein each of the plurality of second lumina (3, 33, 43) is in communication with the outside of the catheter by at least one hole (15, 35, 45, 55).

2. The catheter according to claim 1, wherein the hollow structure is formed of:
a first hollow body (17) that forms the first lumen (5); and
a second hollow body (19) that covers an outer periphery of the first hollow body (17) and is connected to the first hollow body (17) by a plurality of partition walls (21) extending in the direction from the proximal end to the distal end, to form the plurality of second lumina (3) between the first hollow body (17) and the second hollow body (19), and
wherein each of the plurality of second lumina (3) is in communication with the outside of the catheter by at least one hole penetrating the second hollow body (19).

3. The catheter according to claim 1, wherein the hollow structure is formed of:
a hollow body (37) that forms the first lumen (5); and
a plurality of tube bodies (31, 41, 51) disposed along a longitudinal direction of the catheter on the outer periphery of the hollow body (37) to form the plurality of second lumina (33, 43), and
wherein each of the plurality of second lumina (33, 43) is in communication with the outside of the catheter by at least one hole (35, 45, 55) penetrating a respective one of the tube bodies (31, 41, 51).

4. The catheter according to claim 3, wherein the tube bodies (41, 51) are helically wound around the outer periphery of the hollow body (37).

5. The catheter according to anyone of claims 1 to 4, comprising:
a catheter shaft (10, 30, 40, 50) and
a distal end tip (60) provided at a distal end of the catheter shaft (10, 30, 40, 50),
wherein the hollow structure is formed in the catheter shaft (10, 30, 40, 50).
